# EUROPEAN PATENT APPLICATION

(11) **EP 4 789 692 A1**
(43) Date of publication of application: **12.08.2026**
(21) Application number: 24874127.4
(22) Date of filing: 25.09.2024
(51) Int. Cl.: A61L 27/32, A61L 27/34

(54) **ADHESIVE COATING INTENDED FOR TISSUE ENGINEERING AND BONE REGENERATION**

(30) Priority: 02.10.2023 ES 202330826
(71) Applicant: Universidad de Cádiz, 11003 Cádiz Cádiz (ES)
(72) Inventor: SUFFO PINO, Miguel, 11519 Puerto Real Cádiz (ES); CAUQUI LÓPEZ, Miguel Ángel, 11519 Puerto Real Cádiz (ES); PÉREZ MUÑOZ, Celia, 11519 Puerto Real Cádiz (ES); GOMA JIMÉNEZ, Daniel, 11519 Puerto Real Cádiz (ES)
(74) Representative: Pons IP
(86) International application number: PCT/ES2024/070584
(87) International publication number: WO 2025/074017

(57) **Abstract**

The present invention relates to an adhesive coating intended for tissue engineering and bone regeneration that comprises hydroxyapatite (HA) synthesised from Carbocal from the sugar industry and having strong binding mediated by a commercial medical-grade epoxy adhesive. The adhesive coating is an osteoinductive composite material with biocompatible characteristics as of the first 24 hours after use on implantable surfaces or as independent bone filler. In the latter case, it is a material that solidifies in less than three hours and, given that it exhibits mesopores throughout the volume thereof, accepts blood infiltration from the patient who is being surgically operated on.

## Description

### TECHNICAL SECTOR

The present invention belongs to the field of adhesive technology, of chemical engineering and biomedical engineering.

The technical subject matter consists of a biomaterial for the priming process of metallic and non-metallic implantable surfaces, formed by a mixture of an epoxy-type adhesive and hydroxyapatite (HA) obtained from a byproduct of sugar beet processing, as well as for application as an adhesive bone filler, that allows providing osteoinductive mass and a protective barrier for implantable metallic prostheses in the biomedical, traumatology, orthopaedic and oral health industries.

### BACKGROUND OF THE INVENTION

Any foreign body in the organism promotes the development of infections and, accordingly, a rejection of same (Wood et al., 2015). Particularly, this phenomenon is common in dental implants, which are coated by saliva the glycoproteins of which favour colonisation by microorganisms (especially, streptococcus), which proliferate in the oral cavity (Siqueira et al., 2012); (Subramani et al., 2009).

A similar situation occurs with prostheses in traumatology (for example: in total knee arthroplasty, TKA, or THA hip), the used materials of which have been based on stainless steels, titanium alloys, Cr-Co-Ni alloys, Vitalium^{®} and other metals hazardous to humans (Keegan et al., 2007). In this case, the problem arises from a lack of proven biocompatibility in the medium or long term. These parts are designed to have an increased lifespan, to facilitate the integration thereof into the bone by incorporating onto the metallic implantable surfaces a cemented material that serves as a real osteoinductive barrier and protects against infections. Despite achieving a high success rate in these interventions, there is still 19% of patients who express dissatisfaction with the interventions they have undergone and have prompted a review (Bourne et al., 2010).

The literature has described coating methods based on the application of zirconium or aluminium nanoparticles to improve the osseointegration of implants (Salou et al., 2015), even with antimicrobial nanoparticles of Chlorhexidine-hexametaphosphate (CHX-HMP). Said nanoparticles promote good biological fixation in implants and enhance the anti-bacterial effect in the bone (Wood et al., 2015). In fact, most failures in this type of implant are due to poor stability of the piece caused by bone loss that makes it unstable. It is possible to find references that use bioactive glass nanoparticles through calcium precursors using Sol-Gel techniques (Firzok et al., 2019). Furthermore, abrasive treatment processes have been described on the metallic surfaces of implants to make them rougher (1-2 µm) and, in this sense, more osteointegrable but results are debatable and these abrasives leave particles deposited on the implants that behave as contaminants (Schupbach et al., 2019).

Similarly, adhesives have been used to improve screw-implant and screw-abutment (interface) threaded connections (Bosqué, C.;Azevedo, A.D.; Arenas, V.A.; Ávila-Campos, 2019) (Suffo, Vilches-Prez, & Salido-Peracaula, 2020). So that, on the one hand, the thread tightening in the area of the threads where they may not make contact is improved, and at the top where the screw head sits against the prosthetic abutment, the adhesive thus acting as a retainer for the threaded joint. In this manner, the entry of bacteria that induce pathologies is prevented (Caldas et al., 2019).

Moreover, the sugar industry derived from beets, regardless of the variety thereof (Mahmoud et al., 2018), generates high volumes of byproducts/waste that reach 80% of the original raw material (Nanou & Fuentes, 2002). Approximately 140g of sugar can be obtained from each tonne of beetroot, the rest being byproducts/recoverable waste (Canales et al., 2004). These byproducts that are suitable for recovery by forming part of other added value products are: "*Carbocal^{®}", "dried pulp" and, "beet greens" (plant matter).* Currently, these byproducts/waste can be used as animal feed, even in the production of cement for the construction sector, but a large fraction thereof usually end up in landfills.

The scientific literature provides different studies that validate the use of a good number of agricultural wastes as additives or reinforcements of synthetic thermoplastics for different industrial sectors (Ramesh et al., 2017), (Väisänen et al., 2016), (Saba et al., 2016). Among the most commonly used additives in the updated literature, it is worth mentioning: calcium carbonate (CaCO₃), glass fibres, talc, kaolin, mica, wollastonite, silica, graphite, synthetic fillers (e.g., PET or PVA-based fibres), high-performance fibres (carbon, aramid, etc.) (Özen & 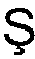 im 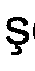 ek, 2015), and reinforcing fibres (hemp, jute, sisal, kenaf, bamboo, etc.) (Gurunathan et al., 2015), (Valsánen et al., 2016). Additionally, patent records have been found that mix dried pulp into polymer matrices for the manufacture of packaging compounds in the food industry [WO2011114101A1], [EP0506650A2], [US2017015820A1], [WO2014074086A1], [AU2017200475A1]. However, no bibliographic references nor registered patents were found relating to the use of Carbocal to ensure a homogeneous mixture with an adhesive resin to obtain a biomaterial for priming implantable surfaces, by way of a protective barrier for those surfaces. Moreover, the reference (Suffo, Mata, et al., 2020) describes the manufacture of an agrocomposite^{®} from Carbocal^{®} and a synthetic thermoplastic for the food industry.

A previous study (Boyetey et al., 2023), discloses the production of a hydroxyapatite derived from the filter cake of sugar cane. This cake has properties similar to Carbocal, however, the latter has a higher percentage of CaCO₃. Furthermore, the process described in this specification, uses as a source of phosphorus: Sodium dihydrogen phosphate monohydrate, whereas in the previous reference they use orthophosphoric acid. Lastly, the hydroxyapatite obtained in said reference is produced through a precipitation process, while that which is presented here is obtained through a phosphatisation process.

The Keller & Bohacek GmbH patent, 1988, ES2003166 (A6), discloses a process for obtaining enzymes from beetroot peelings, but there is no further scientific contribution to the recovery of byproducts/waste, nor as part of the aforementioned composite mixtures. Furthermore, in the scientific literature there are numerous studies of CaCO₃ (the main basic constituent of Carbocal), as an additive for thermoplastics such as (R.N. Rothon, 2003), (Özen & 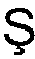 im 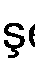 ek, 2015). Lastly, the reference (Kashyap & Datta, 2017) conducts a feasibility study of CaCO₃-rich waste from the paper industry, as a filler for thermoplastics, justifying the improvement of the original properties of the polymer matrix.

From the first studies carried out by (Roy et al., 1974), it was verified how nature has provided biomaterials that, through a physical-chemical treatment, biocompatible structured compounds such as HA have been generated (Tampieri et al., 2009) (Parfen'eva et al., 2006), in a temperature range between 100-1000 °C, between 14-140 MPa of pressure, and a pH between 5 and 12. The reference (Ruffini et al., 2013), transforms rattan wood into HA in a 96-hour long process, with a basic pH and at low temperatures (20-60 °C). Through hydrothermal synthesis, the well-known chemical reaction of phosphatisation of CaCO₃ gives rise to bioceramic structures of calcium phosphate (HA), beta-tricalcium phosphate (β-TCP), and biphasic calcium phosphate (BCP), respecting a certain temperature range, pressures and pH; also known in the literature (Roy & Linnehan, 1974), (Roy et al., 1974). According to the reference (Yoshimura et al., 2004), the best mechanism for HA formation from CaCO₃ is precipitation/dissolution. These compounds are traded at a high market price, approaching €400/100g, and depending on the steps used in the manufacture thereof, it will add even more economic cost to the final compound, as well as the energy consumed throughout the process. Furthermore, currently, clinical practice requires a high amount of bone filling, performing approximately 6,000 such procedures per year, only in Andalusia, which comes at a high cost. However, Carbocal^{®}, as described earlier, as it is a byproduct from the processing of beet sugar, has a low market price and contains more than 80% CaCO₃, apart from some trace elements that make it more attractive for use in biomedical applications, and no patent records relating to such applications have been found, nor as part of an adhesive mixture for priming implantable surfaces.

Similarly, adhesive products for medicinal use based on cyanoacrylates have been registered [US2293969A], [US2467927A], [US2768109A]; [US5684042]; [US3483870]; [US5580565]; [US4764377]; [US4892736]; [US4940579]; [US5,254,132]; [WO 01/32319 A2], with a longer or shorter polymerisation time and, forming compounds together with therapeutic agents or antibiotics. There are also acrylics, and finally epoxy resins [WO2019181721 (A1)]. In the aforementioned reference (Suffo, Vilches-Pérez, et al., 2020) a comparison is established of the adhesion capacity exhibited by some commercial adhesives for medical use in dental applications. In said reference, it was concluded that the adhesive called LOCTITE M31CL exhibited the best performance in terms of adhesion strength on materials used in additive manufacturing (3D printing) such as ULTEM1010^{™}, a biocompatible rigid thermoplastic from the PEI (Polyeteremide) family. In [US6455064B1], a method is described for applying a polymerisable adhesive monomer onto a biologically active drug that acts as an initiator of the adhesive polymerisation. The result is an adhesive compound that is applied to wounds as a substitute for sutures. Since the 1960s, acrylic cements and those based on polymethyl methacrylate (PMMA) mixtures, which polymerise quickly and are applied directly onto the implant itself, are used. Unfortunately, bone removal processes are common in areas where prostheses made of these materials have been implanted, mainly caused by corrosion phenomena.

The surface of the implant is considered to be that which will be in direct contact with the oral tissues, either the soft tissues (gum and connective tissue), or the hard ones (bone). The biology of both is completely different, so the part of the implant that has to come into contact with the same has been modified to allow a greater interrelationship therebetween. Thus, the surface of implants can be classified as smooth or treated. The smooth one is the one that has not undergone any processing other than that of the manufacturing process itself, from the turning of the bar. It is almost flat, with only the roughness caused by the passage of the bur and has a lesser impact on bone formation. It is used so that it is the one that is in contact with the soft tissues, in order to obtain a possible union with the same and an area that is easier to clean. The treated one, on the contrary, is uneven and seeks attachment to bone tissue. The resulting pattern will depend on how the smooth surface is modified to reach same, which can be done with addition methods (plasma, material coating, anodisation, ...), subtraction (shot blasting, acid attack, laser, etc.), oxidation or mixed (combination of vapours). Implants obtained through sintering (metal 3D printing) always have a rough surface finish and must be reworked to smooth same. In the case of coatings, these are usually porous, hydroxyapatite (HA) based, performed by diffusion and by plasma spraying (Heimann, 2016). Among other technologies developed to modify surfaces and coat metallic implants, chemical and electrochemical treatments, such as electrophoresis deposition (Balamurugan et al., 2009), stand out, thermal spraying techniques (Cañas et al., 2016) and the sol-gel method (Sergi et al., 2020). Unfortunately, some of these technologies have drawbacks, such as a weak binding between implants and coatings, modification of the properties of the metallic implant or the coating and the presence of impurities. Therefore, for example, reference [RU2684617C1], discloses a method for applying a bioactive coating to titanium implants, the coating is an electrolyte composed of orthophosphoric acid, hydroxyapatite (HA, Ca₅(PO₄)₃(OH)), germanium and distilled water. The reference (Jun et al., 2010) relates to a hybrid coating material consisting of a silicon and chitosan xerogel for coating titanium implant surfaces. In addition, (Kim et al., 2015) disclose a method for obtaining material based on a silica aerogel with an epoxy resin, whereas, (Guzel Kaya et al., 2018) describe the synthesis of a material based on a silica xerogel with an epoxy resin.

More recently, (Palla-Rubio et al., 2019) perform a sol-gel coating on titanium implants based on silica-chitosan by means of an immersion process (dipcoating). Nevertheless, to achieve a homogeneous solution, small amounts of chitosan are needed, since as the concentration of the biopolymer increases, the solution becomes too viscous and difficult to adhere.

Lastly, (Reyes-Peces et al., 2020), relate to a material for bone regeneration, consisting of a TEOS aerogel, chitosan (4-20% w/w chitosan) and glycidopropoxymethyltriethoxysilane (GPTMS), obtained by means of sol-gel synthesis. Additionally, the reference (Pérez-Moreno et al., 2020) discloses the synthesis and method of an 8% silicon (TEOS) and chitosan hybrid aerogel for use thereof in tissue engineering. As a result of these last two projects, the recent patent P202130841 (Suffo, M.; Piñero, M.; Salido M.; Fernández-Montesinos R.; Reyes-Peces M.V.;De la Rosa, 2021) was applied for, in which a hybrid adhesive/sol-gel coating material is described, characterised in that it comprises a sol-gel material (aerogel or xerogel), made up of tetraethyl orthosilicate (TEOS) and chitosan (4-20% by weight), with a strong binding mediated by a commercial medical-grade epoxy adhesive, mixed in specific proportions for application on metallic and non-metallic implantable surfaces, in tissue engineering and biomechanics. In the explanation of the invention of said patent, bioactivity is demonstrated by introducing the samples into simulated biological fluid and verifying that after 7 days HA grows on the surface thereof. Said patent demonstrates a clear invention, but the manufacturing process of the sol-gel described is laborious and costly, in addition to the fact that it provides no innovation other than hybridisation with the adhesive in a solid bond, since the manufacture of aerogels is well known in the literature. However, no cross-cut tests or trials according to the UNE-EN ISO 2409 standard are indicated, which confirms correct fixation of the coating on the implantable surface. Nor are the sizes and morphologies of the crystalline structures of HA formed specified, considering that micro/nano crystalline structures with a Ca/P ratio of 1.67 are the most suitable for manufacturing biomimetic biomaterials similar to human bone (Lin et al., 2014).

Additionally, the consulted bibliography does not report cases of obtaining a biomaterial for priming metallic or non-metallic implantable surfaces consisting of a mixture of HA and an epoxy resin-based adhesive, such as that described in this invention. This biomaterial potentially plays a bioactive role, exhibiting the ability to induce cell nucleation and growth on the surface of the biomaterial, regardless of whether it is metal or non-metal. Additionally, no references have been found that mention HA synthesised from byproducts of sugar beet treatment.

The adhesive hybrid biomaterials and biocomposites resulting from the processing described in this specification avoid the main problems that arise with implants based on metallic materials such as stainless steel, titanium or chromium-cobalt-nickel alloys, which do not guarantee bone formation and cause problems in the vicinity of the surgery, and above all, corrosion phenomena.

Current prostheses used in traumatology and dentistry, in general, are not designed to receive a coating such as that which is described in this specification. At most, the only thing they use is bone cement as a protective barrier for the metallic material. Tests carried out on metallic and non-metallic surfaces designed to test the effectiveness of the coating showed similar results.

The invention demonstrates the possibility of coating non-bioactive and non-biodegradable metallic surfaces in implantable elements with these characteristics, but with the guarantee of not suffering from detachments.

### Bibliographic references used

Balamurugan, A., Balossier, G., Michel, J., & Ferreira, J. M. F. (2009). Electrochemical and structural evaluation of functionally graded bioglass-apatite composites electrophoretically deposited onto Ti6AI4V alloy. Electrochimica Acta, 54(4), 1192-1198. https://doi.org/10.1016/j.electacta.2008.08.055 https://doi.Org/10.1016/j.electacta.2008.08.055
Bosqué, C.;Azevedo, A.D.; Arenas, V.A.; Ávila-Campos, M. J. (2019). Efficacy of a Polyglycol Dimethacrylate-Based Adhesive in Sealing the Implant-Abutment Interface. Implant Dentistry, 28(3), 265-271.
Bourne, R. B., Chesworth, B. M., Davis, A. M., Mahomed, N. N., Charron, K. D. J., & Met, D. (2010). Patient Satisfaction after Total Knee Arthroplasty Who is Satisfied and Who is Not? Clin. Orthop. Relat. Res., 57-63. https://doi.org/10.1007/s11999- 009-1119-9 https://doi.org/10.1007/s11999-009-1119-9
Boyetey, M. J., Torgbo, S., Sukyai, P., Watthanasakphuban, N., & Kamonsutthipaijit, N. (2023). Filter cake-derived calcium carbonate polymorphs from sugar refinery for hydroxyapatite production as a sustainable material for biomedical application, Ceramics International. 49:14. https://doi.org/10.1016/j.ceramint.2023.04.174. https://doi.Org/10.1016/j.ceramint.2023.04.174
Caldas, I. P., Alves, G. G., Barbosa, I. B., Scelza, P., de Noronha, F., & Scelza, M. Z. (2019). In vitro cytotoxicity of dental adhesives: A systematic review. Dental Materials, 35(2), 195-205. https://doi.org/10.1016/j.dental.2018.11.028 https://doi.Org/10.1016/j.dental.2018.11.028
Canales, C., Cortés, V., & Martínez, J. A. (2004). Guía de mejores técnicas disponibles en España del sector refino de petróleo.
Cañas, E., Vicent, M., Bannier, E., Carpió, P., Orts, M. J., & Sánchez, E. (2016). Effect of particle size on Processing of bioactive glass powder for atmospheric plasma spraying. Journal of the European Ceramic Society, 36(3), 837-845. https://doi.org/10.1016/j.jeurceramsoc.2015.09.039
Firzok, H., Zahid, S., Asad, S., Manzoor, F., Khan, A. S., & Shah, A. T. (2019). Sol-gel derived fluoridated and non-fluoridated bioactive glass ceramics-based dental adhesives: Compositional effect on re-mineralization around orthodontic brackets. Journal of Non-Crystalline Solids, 521(April). https://doi.org/10.1016/j.jnoncrysol.2019.119469
Gurunathan, T., Mohanty, S., & Nayak, S. K. (2015). A review of the recent developments in biocomposites based on natural fibres and their application perspectives. Composites Part A: Applied Science and Manufacturing, 77, 1-25. https://doi.org/10.1016/j.compositesa.2015.06.007 https://doi.Org/10.1016/j.compositesa.2015.06.007
Guzel Kaya, G., Yilmaz, E., & Deveci, H. (2018). Sustainable nanocomposites of epoxy and silica xerogel synthesized from corn stalk ash: Enhanced thermal and acoustic insulation performance. Composites Part B: Engineering, 150(April), 1-6. https://doi.org/10.1016/j.compositesb.2018.05.039https://doi.Org/10.1016/j .compositesb.2018.05.039
Heimann, R. B. (2016). Plasma-Sprayed Hydroxylapatite-Based Coatings: Chemical, Mechanical, Microstructural, and Biomedical Properties. Journal of Thermal Spray Technology, 25(5), 827-850. https://doi.org/10.1007/s11666-016-0421-9
Jun, S. H., Lee, E. J., Yook, S. W., Kim, H. E., Kim, H. W., & Koh, Y. H. (2010). A bioactive coating of a silica xerogel/chitosan hybrid on titanium by a room temperature sol-gel process. Acta Biomaterialia, 6(1), 302-307. https://doi.org/10.1016/j.actbio.2009.06.024https://doi.Org/10.1016/j.actbio .2009.06.024
Kashyap, S., & Datta, D. (2017). Reusing industrial lime sludge waste as a filler in polymeric composites. Materials Today: Proceedings, 4(2), 2946-2955. https://doi.org/10.1016/j.matpr.2017.02.176
Keegan, G. M., Learmonth, I. D., & Case, C. P. (2007). Orthopaedic metals and their potential toxicity in the arthroplasty patient. A review of current knowledge and future strategies. Journal of Bone and Joint Surgery - Series B, 89(5), 567-573. https://doi.org/10.1302/0301 -620X.89B5.18903
Kim, H. M., Kim, H. S., Kim, S. Y., & Youn, J. R. (2015). Silica aerogel/epoxy composites with preserved aerogel pores and low thermal conductivity. E-Polymers, 15(2), 111-117. https://doi.org/10.1515/epoly-2014-0165
Lin, K., Wu, C., & Chang, J. (2014). Advances in synthesis of calcium phosphate crystals with controlled size and shape. Acta Biomaterialia, 10(10), 4071-4102. https://doi.org/10.1016/j.actbio.2014.06.017 https://doi.Org/10.1016/j.actbio.2014.06.017
Mahmoud, E.-S. A., Hassanin, M. A., Borham, T. I., & Emara, E. I. R. (2018). Tolerance of some sugar beet varieties to water stress. Agricultural Water Management, 201, 144-151. https://doi.org/10.1016/J.AGWAT.2018.01.024 https://doi.Org/10.1016/J.AGWAT.2018.01.024
Nanou, C. P., & Fuentes, L. De. (2002). Awarenet: Agro-Food Wastes Minimisation and Reduction Network. Waste Management and the Environment, 1877-1880. https://doi.org/10.2495/WM020321
Özen, I., & 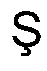 im 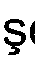 ek, S. (2015). Vital importance of moisture level in all stages of Processing from calcium carbonate coating through polyethylene/calcium carbonate compounding to film generation. *Powder Technology,* 270(Part A), 320-328. https://doi.org/10.1016/j.powtec.2014.10.038 https://doi.Org/10.1016/j.powtec.2014.10.038
Palla-Rubio, B., Araújo-Gomes, N., Fernández-Gutiérrez, M., Rojo, L., Suay, J., Gurruchaga, M., & Goñi, I. (2019). Synthesis and characterization of silica-chitosan hybrid materials as antibacterial coatings for titanium implants. Carbohydrate Polymers, 203(May 2018), 331-341. https://doi.org/10.1016/j.carbpol.2018.09.064 https://doi.Org/10.1016/j.carbpol.2018.09.064
Parfen'eva, L. S., Orlova, T. S., Kartenko, N. F., Sharenkova, N. V, Smirnov, B. I., Smirnov, I. A., Misiorek, H., Jezowski, A., Mucha, J., de Arellano-Lopez, A. R., Martinez-Fernandez, J., & Varela-Feria, F. M. (2006). Thermal and electrical properties of a white-eucalyptus carbon preform for SIC/SI ecoceramics. Physics ofthe Solid State, 48(3), 441-446. https://doi.org/10.1134/S1063783406030061
Perez-Moreno, A., Reyes-Peces, M. de las V., de los Santos, D. M., Pinaglia-Tobaruela, G., de la Orden, E., Vilches-Pérez, J. I., Salido, M., Piñero, M., & de la Rosa-Fox, N. (2020). Hydroxyl groups induce bioactivity in silica/chitosan aerogels designed for bone tissue engineering. In vitro model for the assessment of osteoblasts behavior. Polymers, 12(12), 1-22. https://doi.org/10.3390/polym12122802https://dol.org/10.3390/polym12122 802
R.N. Rothon. (2003). Particulate-filled Polymer Composites, Second Edition. Smithers Rapra Press; 2 edition (January 1, 2003).
Ramesh, M., Palanikumar, K., & Reddy, K. H. (2017). Plant fibre based biocomposites: Sustainable and renewable green materials. Renewable and Sustainable Energy Reviews, 79(February), 558-584. https://doi.org/10.1016/j.rser.2017.05.094
Reyes-Peces, M. V, Pérez-Moreno, A., de-los-Santos, D. M., Mesa-Díaz, M. del M., Pinaglia-Tobaruela, G., Vilches-Pérez, J. I., Fernández-Montesinos, R., Salido, M., de la Rosa-Fox, N., & Piñero, M. (2020). Chitosan-GPTMS-Silica Hybrid Mesoporous Aerogels for Bone Tissue Engineering. Polymers, 12(11). https://doi.org/10.3390/polym12112723 https://dol.org/10.3390/polym12112723
Roy, D. M., Eysel, W., & Dinger, D. R. (1974). Hydrothermal synthesis of various carbonate containing calcium hydroxyapatites. Materials Research Bulletin, 9, 35-39.
Roy, D. M., & Linnehan, S. K. (1974). Hydroxyapatite formed from Coral Skeletal Carbonate by Hydrothermal Exchange. Nature, 247(5438), 220-222. https://doi.org/10.1038/247220a0
Ruffini, A., Sprio, S., & Tampieri, A. (2013). Study of the hydrothermal transformation of wood-derived calcium carbonate into 3D hierarchically organized hydroxyapatite. Chemical Engineering Journal, 217, 150-158. https://doi.org/https://dol.org/10.1016/j.cej.2012.11.107 https://dol.Org/https://dol.org/10.1016/j.cej.2012.11.107
Saba, N., Jawaid, M., Alothman, O. Y., & Paridah, M. T. (2016). A review on dynamic mechanical properties of natural fibre reinforced polymer composites. Construction and Building Materials, 106, 149-159. https://doi.org/10.1016/j.conbuildmat.2015.12.075 https://doi.Org/10.1016/j.conbuildmat.2015.12.075
Salou, L., Hoornaert, A., Louarn, G., & Layrolle, P. (2015). Enhanced osseointegration of titanium implants with nanostructured surfaces: An experimental study in rabbits Acta Biomaterialia Enhanced osseointegration of titanium implants with nanostructured surfaces: An experimental study in rabbits. January 2018. https://doi.org/10.1016/j.actbio.2014.10.017 https://doi.Org/10.1016/j.actbio.2014.10.017
Schupbach, P., Glauser, R., & Bauer, S. (2019). AI2O3 Particles on Titanium Dental Implant Systems following Sandblasting and Acid-Etching Process. International Journal of Biomaterials, 2019, 9-12. https://doi.org/10.1155/2019/6318429
Sergi, R., Bellucci, D., & Cannillo, V. (2020). A comprehensive review of bioactive glass coatings: State of the art, challenges and future perspectives. Coatings, 10(8). https://doi.org/10.3390/COATINGS10080757
Siqueira, W. L., Custodio, W., & McDonald, E. E. (2012). New insights into the composition and functions of the acquired enamel pellicle. Journal of Dental Research, 91(12), 1110-1118. https://doi.org/10.1177/0022034512462578
Subramani, K., Jung, R. E., Molenberg, A., & Hammerle, C. H. F. (2009). Biofilm on dental implants: a review of the literature. The International Journal of Oral & Maxillofacial Implants, 24(4), 616-626. https://doi.org/10.5167/uzh-26110
Suffo, M.; Piñero, M.; Salido M.;Fernández-Montesinos R.;Reyes-Peces M.V.;De la Rosa, N. (2021). Recubrimiento híbrido basado en una mezcla de material compuesto adhesivo, para aplicación en superficies de elementos implantares (Spain: P202130841). (Patent No. P202130841).
Suffo, M., Mata, M. D. L., & Molina, S. I. (2020). A sugar-beet waste based thermoplastic agro-composite as substitute for raw materials. Journal of Cleaner Production, 257. https://doi.org/10.1016/j.jclepro.2020.120382https://doi.Org/10.1016/j.jclep ro.2020.120382
Suffo, M., Vilches-Pérez, J. I., & Salido-Peracaula, M. (2020). Comparative Analysis of the Adhesion of Metallic Inserts on Dental Implants-Prosthetic Assembly Generated by Polymeric Materials Used for Additive Manufacturing. In Advances in Design Engineering; Lecture Notes in Mechanical Engineering; Springer: Berlin, Germany (Vol. 1, pp. 245-253). https://doi.org/10.1007/978-3-030-41200-5_27
Tampieri, A., Sprio, S., Ruffini, A., Celotti, G., Lesci, I. G., & Roveri, N. (2009). From wood to bone: Multi-step process to convert wood hierarchical structures into biomimetic hydroxyapatite scaffolds for bone tissue engineering. Journal of Materials Chemistry, 19(28), 4973-4980. https://doi.org/10.1039/b900333a
Väisänen, T., Haapala, A., Lappalainen, R., & Tomppo, L. (2016). Utilization of agricultural and forest industry waste and residues in natural fiber-polymer composites: A review. Waste Management, 54, 62-73. https://doi.org/10.1016/j.wasman.2016.04.037 https://dol.Org/10.1016/j.wasman.2016.04.037
Wood, N. J., Jenkinson, H. F., Davis, S. A., Mann, S., O'Sullivan, D. J., & Barbour, M. E. (2015). Chlorhexidine hexametaphosphate nanoparticles as a novel antimicrobial coating for dental implants. Journal of Materials Science: Materials in Medicine, 26(6), 1-10. https://doi.org/10.1007/s10856-015-5532-1 https://dol.org/10.1007/s10856-015-5532-1
Yoshimura, M., Sujaridworakun, P., Koh, F., Fujiwara, T., Pongkao, D., & Ahniyaz, A. (2004). Hydrothermal conversion of calcite crystals to hydroxyapatite. Materials Science and Engineering C, 24(4), 521-525. https://doi.org/10.1016/j.msec.2004.01.005 https://doi.Org/10.1016/j.msec.2004.01.005

### DESCRIPTION OF THE INVENTION

The invention develops a biomaterial comprising hydroxyapatite (HA) synthesised from a byproduct of sugar beet processing known as Carbocal, with a strong binding mediated by a commercial medical-grade epoxy adhesive, for use thereof in primers that prepare implantable surfaces to make the same osteoinductive.

The bioactivity of the biomaterial is demonstrated by means of a cytotoxicity assay, in which cell growth is demonstrated at almost the same level as the positive control on the surface thereof, within the first 24 hours. The correct adhesion of the biomaterial for proper priming is demonstrated by the cross-cut test according to the UNE-EN ISO 2409 standard. Therefore, a biomaterial for priming implantable surfaces is achieved that complies with the three basic requirements for osteoinduction: biocompatible, bioactive and, biodegradable. This biomaterial is used in tissue engineering as a scaffold and also as an adhesive filler that promotes bone regeneration.

The proposed surface priming biomaterial, which is the result of mixing HA synthesised from Carbocal and a commercial medical-grade epoxy resin, begins with the synthesis of HA in a hydrothermal reactor.

The sample prepared from carbocal waste exhibits a biphasic nature that is important within the framework of the concept of bioactivity proposed by Daculsi in France, and Lynch, Nery and LeGero in the USA in the 1980s (Daculsi et al., 1989). As is well known, this concept was based on the unique properties of biphasic calcium phosphate ceramics (so-called BCPs). BCP bioceramics consist of a mixture of hydroxyapatite (HA) (more stable) and beta-tricalcium phosphate (β-TCP) (more soluble), of variable HA/b-TCP ratio. This material, structurally similar to the HAcc prepared in this work, gradually dissolves in the body, generating new bone formation as it releases Ca2+ and PO43- ions into the biological environment. Furthermore, both phases are biocompatible, essential property to avoid adverse reactions and promote integration with the surrounding tissue. BCP is commercially available in Europe, Brazil, Japan, US, Australia as bone graft or bone substitute material for orthopedic and dental applications under various commercial trademarks (BCP^{®}, MBCP^{®}, Triosite^{®}, Hatric^{®}, Eurocer^{®}, Biceram^{®}, Bicalfoss^{®}).

The hydrothermal technique was selected for the synthesis of the material due to the simplicity and speed thereof and, above all, low cost. In this particular case, Carbocal powder will be used, sourced from the owning company (AB Azucarera) as it comes from the sugar refining process. Applying the well-known recipe for hydrothermal phosphatisation of CaCO₃ (Tampieri et al., 2009), (Yoshimura et al., 2004), but using a sodium salt instead of potassium and respecting the molar ratio (CO₃/PO₄ = 5/3), it is carried out under identical conditions.

### BRIEF DESCRIPTION OF THE FIGURES

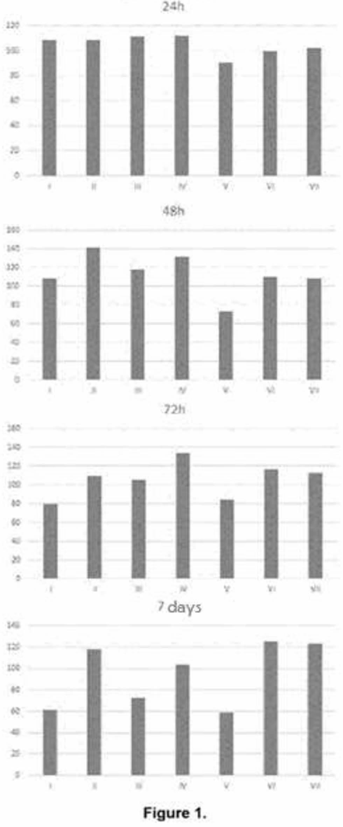

**Figure 1****.** Results obtained in the viability cell assay.

### PREFERRED EMBODIMENT OF THE INVENTION

Two comparative trials are carried out with the same characteristics, in other words, in a 200 ml beaker, 9 grammes of Carbocal (80% w/w) are mixed with 6 grammes of sodium hydroxyphosphate in 100ml of water until complete dissolution of both reagents. At this point, a constant bubbling of CO₂ is observed, a product of the reaction that is taking place, it is left to stir for 2 hours to allow the reaction to complete, and the resulting pH is subsequently measured (approx. 7).

Using respective portions of water, the product is transferred to the 250ml Teflon beaker, reaching a volume of 80% of its nominal capacity (200ml in total), it is sealed in the steel hydrothermal reactor and transferred to the oven at 200 °C for a period of 24 hours. Once cooled to room temperature, the contents of the beakers are extracted, washed, centrifuged (5000rpm for 5min), the remaining liquid is removed and it is dried in an oven at 120°C for 24 hours. Once the material is dry, it is crushed in an agate mortar and sieved through a 75 micron mesh to obtain a homogeneous powder. The result of both tests is identical, and a total of 14, 15 grammes of reaction product are obtained.

The HA obtained has a biphasic nature: 75% w/w HA and 24.83% w/w β-TCP, which is beneficial for the biocompatibility thereof, as explained above. In the FTIR analysis of HA, a peak corresponding to the presence of CO₃ groups which are not present when analysing a commercial HA, is observed, so that the synthesis of HA from Carbocal presents another benefit, since the natural HA present in bones also possesses these CO3 groups.

**Table 1. Density results and specific surface area**

| *Material* | *Density (g cm⁻³)* | *Specific surface area (m² g⁻¹)* |
|---|---|---|
| Synthesised HA | 0.29±0.01 | 30±5 |

Next, the synthesised HA powder is manually mixed with the biocompatible epoxy adhesive until a greenish-brown biomaterial with a homogeneous texture is obtained.

The adhesive used is a medical-grade epoxy called LOCTITE^{®} M31 CLTM from the manufacturer HENKEL IBERICA, S.A. It was selected from among 5 products based on the results obtained from the adhesion test carried out in the aforementioned reference (Suffo, Vilches-Pérez, et al., 2020). It is a two-component adhesive based on a resin and a hardener (2:1 ratio, resin:hardener), the mixture of which results in an almost transparent colour, having low viscosity and a specific gravity of 1.07 kg/cm3 at 25 °C.

The mixture with the adhesive was made following different HA ratios as shown in Table 2, which affects the behaviour thereof. These mixtures are cured in 90 minutes at 30°C and have withstood the autoclave sterilisation process up to 80°C without deformation or phase changes.

**Table 2. Ratios used for the biomaterial**

| Component | Mixture (mg) | | | | | | |
|---|---|---|---|---|---|---|---|
| | I | II | III | IV | V | VI | VII |
| A (narrow tube) | 138.2 | 135.5 | 133.5 | 133 | 275 | 345.3 | 310 |
| B (wide tube) | 266.1 | 276.7 | 275.4 | 264 | 566.1 | 720 | 740 |
| HA | 40.9 | 100 | 199.7 | 300.4 | 843.6 | 1300.5 | 871* |
| | | | | | | | |
| Total | 445.2 | 512.2 | 608.6 | 697.4 | 1684.7 | 2365.8 | 1921 |
| cHA | 9.19 | 19.52 | 32.81 | 43.07 | 50.07 | 54.97 | 45.34* |
| Epoxy | 90.81 | 80.48 | 67.19 | 56.93 | 49.93 | 45.03 | 54.66 |

| | | | | | | | |
|---|---|---|---|---|---|---|---|
| *Use of cHA doped with beet pulp | | | | | | | |

Lastly, cell cultures were performed using human foetal osteoblastic cell lines (hFOB 1.19), in the presence of the generated biomaterials, to determine if the materials affect cell viability. The hFoB (Human Foetal Osteoblasts) cell line is an established cell line, widely used in research related to bone biology and osteogenesis, since it is derived from human foetal bone tissue. Being osteoblasts, in other words, specialised cells in the formation and mineralisation of bone tissue, allows them to be used in research related to bone regeneration.

For this cell viability study, hFOB was cultured in osteogenic media and viability assays were performed at 24, 48, 72 hours and 7 days. In this test, the cells were incubated with MTS and the absorbance was subsequently quantified. As a negative control, before the process, hFOB cultures were incubated with 70% methanol for 30 minutes. Untreated hFOB cultures were used as a positive control.

Normal human foetal osteoblasts were acquired from ATTC (hFOB cell line 1.19, CRL-11372TM). hFOB cultures were grown in osteogenic media containing Dulbecco's modified Eagle culture (DMEM-Gibco), with 10% foetal bovine serum (FBS - Corning) and antibiotic (G418 30 mg/uL - PAN Biotech). The cells were expanded by incubation at 35 °C in 75 cm² flasks with 5% CO₂.

The viability tests were performed over 7 days. As a negative control, before the marking process, hFOB cultures were incubated with 70% methanol for 30 minutes. As a positive control, untreated hFOB cultures were used.

Before the test, the materials were sterilised using an autoclave at 120°C for 20 minutes. For the viability study, the cells were cultured with complete medium in 96 well plates at a concentration of 2 x 10⁴ cells/well. These plates were incubated at 35°C with 5% CO2 for 24 hours. In the case of positive control (PC) and negative control (NC), the potholes were left to grow without adding anything, whereas, in the wells corresponding to the tests, the different biomaterials were added.

To measure viability, the MTS test was used. Before the marking, as a negative control, the cells were incubated with 70% methanol for 30 minutes. 20 ul of MTS was added to all wells and were incubated at 34 °C for 1 hour. After this time, the absorbance was measured at 490nm using the Varioskan LUX plate reader. This process was carried out at 24, 48, 72 hours and 7 days of incubation with the different HA sample solutions. The test was carried out in n = 3 replicates, performing the repetition on 3 different samples.

Furthermore, the LIVE/DEAD cytotoxicity assay was performed, with the aim of determining whether the cells showed growth attached to the surface of the tested biomaterials. To do this, they were grown in the cells under the same conditions described above, on the surface of the materials, for 24 hours. After this time has elapsed, the reagents Calcein (3.5 uM) and EthD-1 (7 uM) were added, and were incubated for 1 hour at 37 °C. For the positive control, the cells were grown without the presence of any biomaterial. For negative control, the same materials were used, treated with the reagents, without cell culture. Biomaterials, as well as controls, were observed under a fluorescence microscope, to determine if there was growth of adherent cells on the surface.

The results obtained in the viability test can be seen in Figure 1, where the cell viability of each material is expressed as a percentage, compared to the positive control, considered as 100%. The negative control never exceeded 30% viability. It is observed that all the ratios used are biocompatible, mixtures VI and VII showing the greatest viability after 7 days. It is observed that, when beet pulp is used as an additive to HA, the required ratio thereof, regarding the epoxy, is lower to achieve the same viability (0.83:1). Therefore, the HA:epoxy ratio range that maintains viability is ≥ 0.10:1 and ≤ 1.22:1.

The LIVE/DEAD assay revealed that cells grow attached only on the VI and VII ratio, which are therefore the most optimal for the established purpose.

### Mode in which the invention is susceptible of industrial application

Currently available bone coatings, are dispensed independently of the prostheses. Commercially available medical-grade epoxy adhesives such as LOCTITE M31CL are dispensed via dual-channel bottles for gun application. The application that is the basis of the present invention in the biomedical sector must be dispensed along with the prosthesis itself, with the appropriate coating dose, or separately in plastic-coated and sterilised envelopes, ready to be applied directly onto an implantable surface or as a bone filler to be applied to the area, accepting a few drops of blood serum prior to application.

## Claims

1. An adhesive coating intended for tissue engineering and bone regeneration, **characterised in that** it comprises hydroxyapatite (HA) synthesised from the sugar industry byproduct Carbocal, biphasic with one HA phase and one TCP phase, having strong binding mediated by a commercial medical-grade epoxy adhesive.

2. The adhesive coating, according to claim 1, **characterised in that** the HA has a biphasic nature: 75% w/w HA and 24.83% w/w β-TCP.

3. The adhesive coating, according to claims 1 and 2, **characterised by** exhibiting cell viability, according to the HA:epoxy adhesive ratio, in the following range: ≥ 0.10:1 and ≤ 1.22:1 (w/w).

4. The adhesive coating, according to claim 3, **characterised by** exhibiting maximum cell viability, according to the HA:epoxy adhesive ratio, at 1.22:1 (w/w).

5. The adhesive coating, according to claim 3, **characterised by** exhibiting maximum cell viability, according to the HA:epoxy adhesive ratio, at 0.83:1 (w/w), when using HA doped with beet pulp.

6. Use of the adhesive coating, according to claims 1 to 5, for application thereof in tissue engineering and biomechanics.

7. Use of the adhesive coating, according to claims 1 to 5, for application thereof on surfaces of implantable elements.

8. Use of the adhesive coating, according to claims 1 to 5, for application thereof in metal prostheses.

9. Use of the adhesive coating, according to claims 1 to 5, for application thereof in non-metallic prostheses.
